# EUROPEAN PATENT SPECIFICATION

(11) **EP 2 538 814 B1**
(45) Date of publication and mention of the grant of the patent: **22.11.2017**
(21) Application number: 11705244.9
(22) Date of filing: 21.02.2011
(51) Int. Cl.: A45D 29/18, A45D 34/06, A61M 35/00

(54) **A NAIL CARE DEVICE**
NAGELPFLEGEVORRICHTUNG
DISPOSITIF DE SOINS DES ONGLES

(30) Priority: 26.02.2010 GB 201003340
(43) Date of publication of application: 02.01.2013
(73) Proprietor: LRC Products Limited, Slough, Berkshire SL1 3UH (GB)
(72) Inventor: ARMSTRONG, Mark, Gwynedd LL41 4PS (GB); CORVIN-CZARNODOLSKI, Timothy, Stanley, Buckinghamshire HP5 2XL (GB); JOHNSON, Neil, Manchester Greater Manchester M41 7HA (GB)
(74) Representative: O'Brien, Niall James
(86) International application number: PCT/GB2011/050333
(87) International publication number: WO 2011/104530

(56) References cited:
- WO-A2-2009/156682
- FR-A1- 2 903 673
- FR-A1- 2 933 282
- GB-A- 2 358 627
- JP-U- H03 123 406
- US-A1- 2004 155 046
- US-A1- 2006 124 147

## Description

The present invention relates to a nail care device. The invention also relates to a nail care device comprising a dispensing mechanism operable to dispense the nail files. The devices of the invention can be used in the therapeutic and non-therapeutic treatment of fingernail and toenail conditions, including but not limited to fungal nail (onychomycosis).

Fungal nail arises due to invasion of the nail bed by dermatophytes species, and manifests itself as yellow, brown or white discoloured patches in the nail. Onychomycosis is a degenerative condition and, if untreated, the invading dermatophytes spread proximally through the nail plate and bed, leading to breakdown and eventual detachment of the nail plate. A person suffering from fungal nail is often concerned with the negative cosmetic impact on the nails and desires to improve the visual appearance of the nails.

It is known to provide a topical treatment for fungal nail, in which a fungal nail care fluid is applied to the nail. Some of the treatments require complete removal of the nail and some of the treatments require the nail bed to be debrided prior to application of the fungal nail care fluid. For example, a product sold under the name ClearZal® BAC, includes a liquid which contains 0.1% w/w benzalkonium chloride, and provides a nail file to debride the nail prior to application of the liquid. Whilst the debridement may increase efficacy, the re-use of the file will promote the spread of the infection to other nails of the same person or to the nails of other people via the transfer of hyphae or spores from the infected nail to a non-infected or recently cured nail. A user can be encouraged to purchase further nail files, but this is an extra inconvenience.

The present invention addresses these drawbacks and aims to provide a device having a convenient supply of nail files.

In accordance with one aspect the present invention provides a nail care device comprising a body provided with a reservoir for a nail care fluid, an applicator for applying the nail care fluid to a nail region, a chamber in the body and a plurality of nail files located in the chamber and removable one at a time therefrom through an outlet opening, wherein the reservoir is formed by a housing, detachably connected to the body. By providing a body with a chamber in which a plurality of nail files can be supplied and stored, and which files can be removed one at a time from the chamber through an outlet opening, the device provides the user with a convenient supply of nail files. Furthermore the user can be encouraged to discard each file after a single use of that file.

Detaching the housing may provide a convenient way of using the device and may further provide a convenient way of accessing fluid inside the housing. The housing may be connected directly or indirectly to the body.

The housing may be connected to the body via a threaded connection. This provides a convenient way of attaching and detaching the housing from the body. Other forms of connection which enable a reservoir housing to be detached from the body are possible, for example a bayonet connection or a magnetic connection.

Different forms of liquid applicator are possible for applying liquid supplied from the reservoir. In some embodiments the applicator may be detachably connected to the body. The applicator may be connected directly or indirectly to the body.

The applicator and reservoir housing may detach as a unit from the body to facilitate application of the nail care fluid. Alternatively the applicator could be separately detachable. If the applicator and the reservoir detach as a unit from the body, the unit may be attached to the body via a form fit connection. In particular, the applicator and the housing may be attached to the body via a collar, which collar is preferably detachable from the body.

Also, the applicator may be detachably connected to the housing. This is advantageous when, for example, the applicator comprises a brush which a user dips into the nail care fluid and uses to transfer the fluid to a nail. The applicator may be connected directly or indirectly to a reservoir housing.

The applicator and reservoir are not limited to any particular form. Rather than a brush and bottle the applicator may alternatively be disposed at an exit point of the reservoir, to receive liquid from the reservoir which may be equipped with a valve or the like to control delivery of liquid. For example, in a manner known per se it may be possible for a user to squeeze the reservoir to dispense the nail care fluid. The applicator may comprise a nozzle through which liquid is applied to a nail, or a spreading element such as a foam pad.

The reservoir may be arranged at one end of the body and the chamber may be arranged at the other end of the body. This provides a convenient configuration for the device which allows fluid in the reservoir to be easily accessed and nail files in the chamber also to be easily accessed.

The nail care device may be a fungal nail care device and the nail care fluid may be fungal nail care fluid. Other forms of nail care device, which is defined in appended claims, are within the scope of the present invention, and may be adapted to achieve both therapeutic and non-therapeutic effects. Of course the device is not limited to applying nail care fluid to a nail and the device could alternatively or additionally be used to apply nail care fluid to another nail region, for example a nail bed.

The nail care device may comprise a dispensing mechanism operable to dispense the nail files one at a time through the outlet opening. This feature advantageously assists removal of the nail files one by one. The present invention provides also a nail care device as defined in claim 1 and comprising a dispensing mechanism operable to dispense the nail files one at a time through the outlet opening. It constitutes an effective arrangement for supplying a number of individual nail files that can be readily dispensed for use one at a time when required.

In one preferred construction the nail files are stacked in the chamber and the dispensing mechanism comprises a biasing means to urge the nail files towards a dispensing position which is a position occupied by a nail file that is ready to be dispensed by operation of the dispensing mechanism. For example the dispensing position may be adjacent the outlet opening.

The dispensing mechanism may comprise an element engageable by a user to cause a nail file to at least partially exit the chamber. This element may be moveable or it may be, for example, an electronic sensor.

In a simple and convenient form the dispensing mechanism can be manually actuable. Thus, the element may be connected to a pushing means for exerting a force against an end of a nail file located in the dispensing position and ready to be dispensed. In particular the dispensing element may be a slide member, moveable towards the outlet opening.

At least five nail files may be accommodated in the chamber. This enables a sufficient number of nail files to be supplied to a user to last at least one month, if one nail file is used per week.

The dispensing device may comprise a blocking means to prevent a user reinserting a nail file through the outlet opening into the chamber. This encourages or minimizes cross-use of nail files. The nail files are intended to be disposed of by a user after they have been used once to debride a nail. By preventing a user from reinserting a used nail file into the chamber a user will not be able to store the used nail file in the device and is encouraged to throw the used nail file away as well as being reminded to use a new nail file next time they carry out a nail treatment. This may be especially important in some applications, for example in the treatment of fungal nail, where a user can re-contaminate nails or pass on the infection to other nails if a nail file is reused. The blocking means may be another nail file still in the device itself, such as in the dispensing position ready to be the next file to be dispensed. The blocking means may additionally or alternatively comprise a biased flap across the outlet opening for example.

The device may be configured to enable the nail files to be replenished. A user can advantageously refill the device when all the nail files have been exhausted from the device. For example, the body may comprise two shells which can be separated by a user to gain access to the chamber to refill it with a fresh supply of nail files.

The nail files may be of conventional form such as board coated with carborundum. The nail files may take any shape and size and the device will be configured accordingly.

The nail care device may be configured to enable the fluid in the reservoir to be replenished. This may involve replacing the reservoir, or the reservoir could be refilled. The applicator may comprise a punch through means to break a seal to access fluid in the reservoir.
Figure 1 is a perspective view of a nail care device;
Figure 2 is a longitudinal cross-sectional view through the nail care device of Figure 1;
Figure 3 is a longitudinal cross-sectional view through the nail file device of Figure 1 with the housing and the applicator omitted;
Figure 4 is a perspective view of a single nail file;
Figure 5 is a cross-sectional view along the line B-B shown in Figure 3;
Figure 6 is an exploded view of the nail care device of Figure 1;
Figure 7 is a view of the interior of the first shell of the nail care device of Figure 1;
Figure 8 is an underside view of the slide of the nail care device of Figure 1;
Figure 9 is a longitudinal cross-sectional view through the nail care device of Figure 1;
Figure 10 is a longitudinal cross-sectional view through the nail care device of Figure 1;
Figure 11 is a longitudinal cross-sectional view through the nail care device of Figure 1 in which nail files of a maximum thickness are contained in the chamber;
Figure 12 is a longitudinal cross-sectional view through the nail care device of Figure 1 and there are no nail files in the chamber; and
Figure 13 is a view showing the nail care device of Figure 1 partly disassembled.

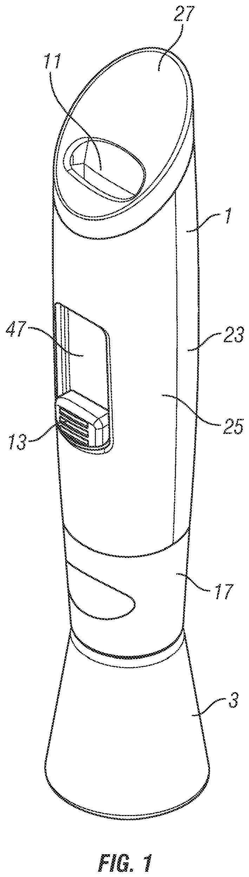

Figure 1 shows an isometric view of a nail care device according to a first embodiment. The device has a body 1 comprising a first shell 23 and a second shell 25 which cooperate with one another. A chamber 7 is provided in the body 1. The body has an end cap 27 which surrounds the first and second shells 23, 25 at a first end of the body. The cap is provided with an outlet opening 11 which is an outlet from the chamber to outside the device.

At a second end of the body, opposite the first end, there is provided a container or housing 3 that serves as a reservoir for a nail care fluid. The nail care fluid in particular is a fungal nail care fluid. The housing 3 is shown to be frustoconical, with a flat base to enable the housing 3 to support the body 1 when positioned on a surface. The housing 3 is fluid-tight and conveniently is made of glass and opaque to ultraviolet radiation.

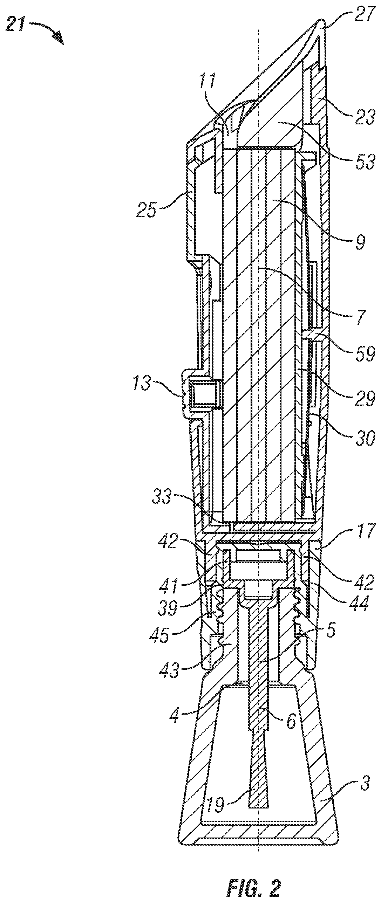

Figure 2 shows a longitudinal cross-sectional through the nail care device of Figure 1. A plurality of nail files 9 are provided in a stack in the chamber 7. The nail files are removable one at a time from the chamber 7 through the outlet opening 11. More especially a dispensing mechanism 21 is provided and is operable to dispense the nail files one at a time through the outlet opening 11. The dispensing mechanism is described in more detail below.

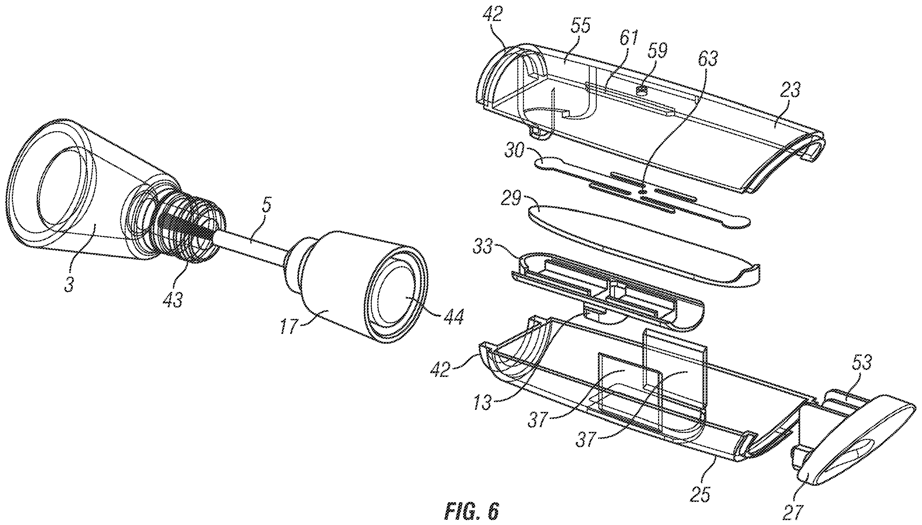

The nail file dispenser is arranged at one end of the nail care device and the housing 3 is arranged at the other end. An applicator 5 for applying nail care fluid to a nail is arranged between the chamber 7 and the housing 3. The applicator 5 is attached to the body 1, and the body is provided with a collar 17 having a form fit connection to the body 1, the applicator 5 being attached to the body 1 via the collar 17. The applicator 5 is attached to the collar 17 by a form fit connection, in which an annular ring 41 of the applicator 5 is received in a complementary circumferential groove 39 on an inner surface of the collar 17. The body shells 23, 25 form an annular skirt 42 that is received in a complementary annular recess 44 formed in the collar 17 (see Figure 6).

The housing 3 is also attached to the body 1 via the collar 17. The housing is detachable from the applicator 1 and a neck of the housing 3 is provided with an external screw thread 43 which co-operates with an internal thread 45 on the collar 17 to form a threaded connection between the housing 3 and the collar 17.

The applicator comprises a brush with a stem 6 and a brush head 19 at one end. The brush is located in the housing 3 when the device is assembled. A sealing element 4 surrounds the stem 6 to seal fluid inside the housing 3 when the device is assembled. The seal may be air tight. The housing thus has a re-sealable access point and the fluid can be accessed through removal of the brush from the housing 3.

To apply nail care fluid to a nail, an operator unscrews the housing 3 to detach the housing 3 from the applicator 5. Fluid in the housing 3 can be accessed and the brush can be used to apply fluid from the housing 3 to a nail. The body 1 may act as a handle for the device when fluid is being applied. Alternatively, the collar 17, the applicator 5 and the housing 3 may be detached from the body 1 and the collar 17 may be used as a handle when fluid is applied. If the fluid in the housing 3 is exhausted, a user can dispose of the housing 3 and employ a new housing 3 charged with fluid with the device.

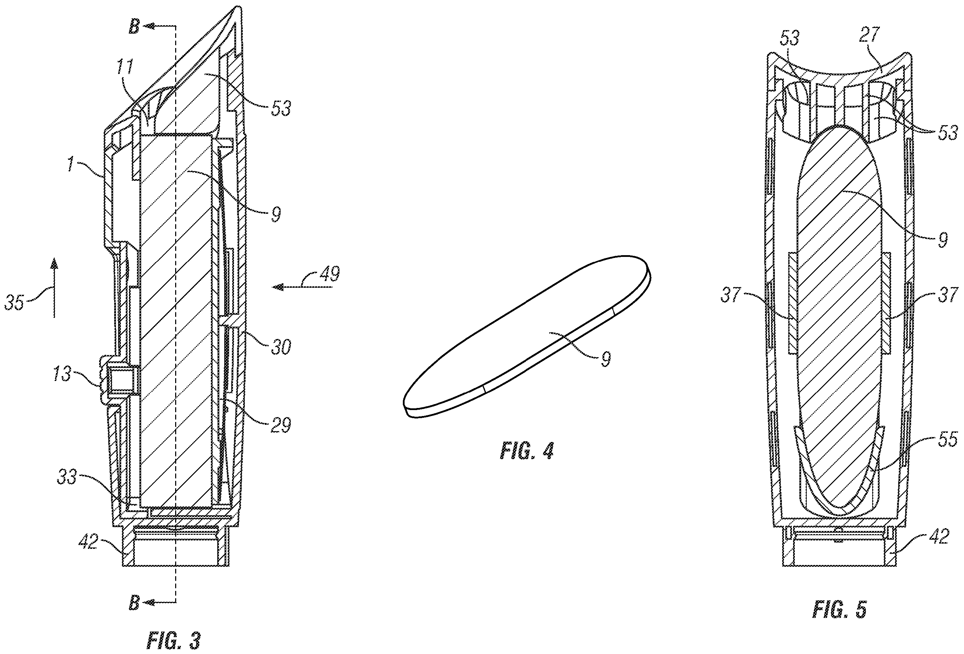

Figure 3 shows a cross-sectional view of the nail file dispenser with the housing 3 and the applicator 5 being omitted. The nail files 9 accommodated in the chamber 7 are located on top of one another in a stack. The uppermost nail file 9 in the stack is the one which will be dispensed first, and the lowermost file 9 is the one which will be dispensed last from the chamber. The lowermost nail file rests against a blank 29. The blank is a similar size and shape to the nail files 9. Figure 4 shows a perspective view of a single nail file 9.

A spring 30 is arranged between the blank 29 and the inner surface of the body 1. The spring exerts a biasing force against the blank 29 to urge the blank 29 and therefore the stack of nail files 9 towards a dispensing position. The dispensing position is the position which a nail file occupies immediately before the dispensing mechanism is operated to dispense that nail file through the outlet opening 11. In the present embodiment, the dispensing position is a position adjacent to and in alignment with the outlet opening 11.

Figure 5 shows a cross-sectional view along the line B-B in Figure 3. The nail files 9 are confined and guided on either side by a side arm 37 to prevent the nail files 9 from moving laterally. The nail files are prevented from moving away from the outlet opening 11 by an end guide 55 which is curved to cup the inner ends of the nail files. In the embodiment shown, the end guide 55 is formed on the first shell 23, and the side arms 37 are formed on the second shell 25 as shown in Figure 6.

Figure 5 further shows a plurality of fins 53 which are formed on the cap 27. The fins act as a stop to prevent movement of the lower nail files towards the outlet opening 11 when the uppermost nail file is being dispensed.

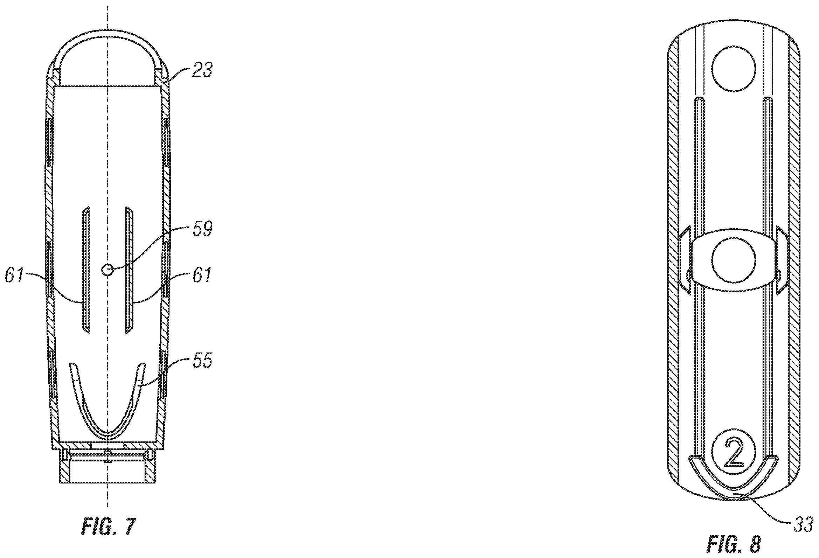

Figure 6 shows the spring 30 when pressed flat. The spring is a leaf spring 30 made of stainless steel which is curved when located inside the device to exert the upward biasing force on the blank 29. The spring 30 has a central hole 63 for attaching the leaf spring 30 to the first shell 23. The first shell 23 has a protrusion 59 which fits into the central hole 63 as may be seen in Figures 2 and 7 to retain the spring in position. Figure 7 shows that the first shell 23 is provided with a pair of parallel spring guide rails 61 which prevent the spring from twisting when it is located on the protrusion 59.

As best seen in Figure 3, the slide 13 is mounted in the body 1 and is movable in a direction indicated by the arrow 35. The slide 13 follows a linear path towards and away from the outlet opening 11. The slide 13 has an actuating button that protrudes from the body 1, the body 1 having an opening 47 through which the button extends and along which the button is moveable during movement of the slide 13 (see Figure 1). The slide 13 is elongated and at its end remote from the outlet 11 has an abutment 33 for engaging against the inner end of the nail file 9 located in the dispensing position.

Figure 8 is an underside view of the slide, as seen in the direction indicated by the arrow 49 in Figure 3. The abutment 33 has a curved shape which may correspond to the curvature at the end of a nail file 9 located in the dispensing position as also shown in Figure 6.

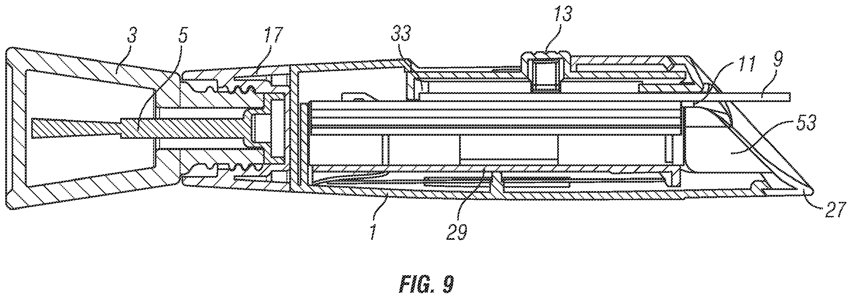

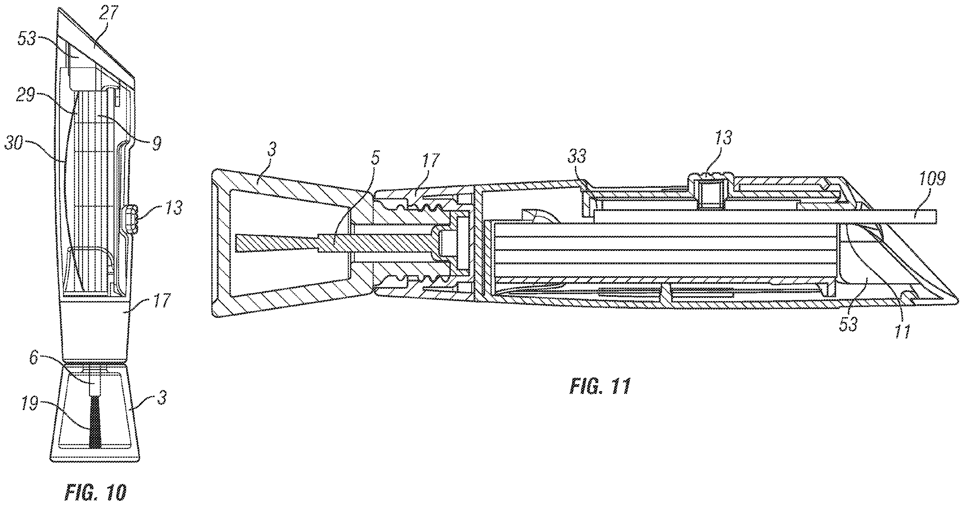

To dispense a nail file, a user pushes on the button to move the slide 13 towards the outlet opening 11. The abutment 33 pushes against the inner end of the nail file 9 located in the dispensing position to move the nail file towards and through the outlet opening 11. The nail file protrudes sufficiently out of the outlet opening 11 for an operator to gain purchase on the file and remove the file entirely from the chamber 7. The fins 53 on the cap act as a stop to prevent the remaining nail files in the stack from moving when the uppermost nail file is being dispensed. Figure 9 shows the dispensing mechanism after the slide 13 has been moved towards outlet opening 11 to its end position for dispensing a nail file. The device contains nail files 9 of a certain thickness. It should be noted that Figure 9 shows the blank 29 in a lower position. In reality the blank 29 would be sitting directly adjacent the stack of nail files, as more accurately represented in Figure 10.

Once a file has been removed, the user can move the slide 13 back away from the outlet opening 11. The abutment 33 prevents a nail file from moving into the dispensing position, until the abutment passes beyond the nail files 9. At this point, the force of the spring 30 causes the remaining nail files 9 to move towards the dispensing position, so that the next file 9 in the stack is located in the dispensing position ready to be next dispensed.

The dispensing mechanism 21 includes a blocking means which prevents a user inserting a nail file into the chamber 7. A user is instructed to move the slide 13 away from the outlet opening 11 after a nail file has been dispensed, which results in a nail file 9 being located in the dispensing position as described above. The nail file 9 located in the dispensing position prevents a user from re-inserting a used nail file through the outlet opening 11 into the chamber 7.

Figure 11 shows the dispensing mechanism after the slide 13 has been moved towards outlet opening 11. The device contains nail files 109 of a greater thickness than the nail files 9 shown in the device in Figure 9. Figure 11 shows the maximum thickness of nail file which can be dispensed from a device since the thickness of the nail files 109 corresponds to the width of the outlet opening 11.

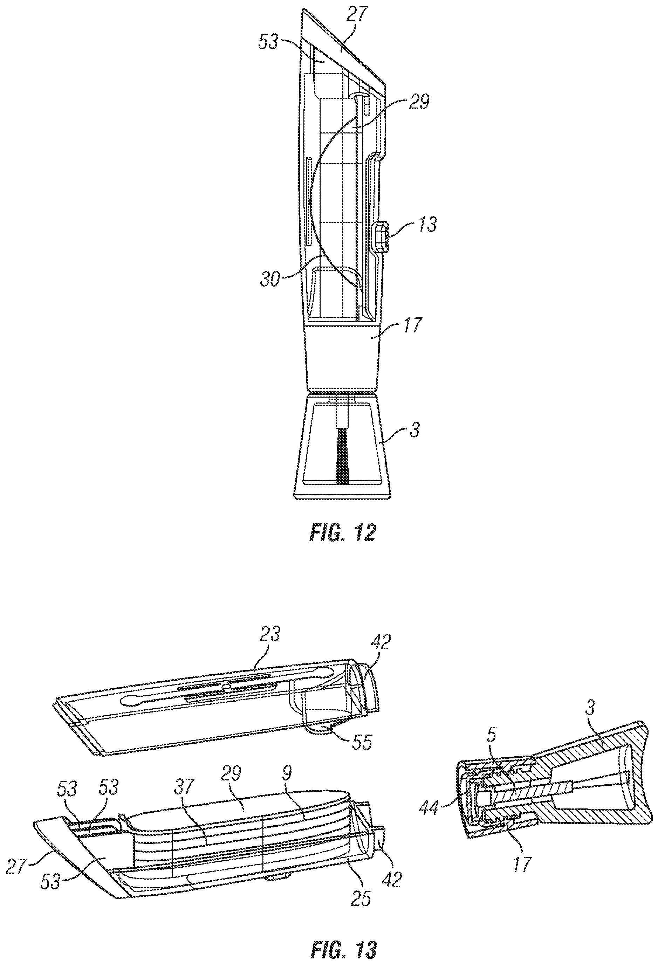

Figure 12 shows the device after all the nail files 9, 109 have been dispersed. The leaf spring 30 still contacts the blank 29, and has a relatively small radius of curvature when compared to its radius of curvature when nail files are located in the device (see Figure 10).

Figure 13 shows the device partly disassembled so that access can be gained to the chamber 7 to allow a fresh supply of nail files to be inserted in the device. The collar 17, the applicator 5 and the housing 3 have been removed from the body 1 so that the first shell 23 can be separated from the second shell 25. This provides access to the chamber 7 into which the nail files 9 are loaded in a stack, one on top of another. The nail files are loaded into the second shell 25 between the side arms 37. The first shell 23 can then be assembled back in place, so that the end guide 55 cups the end of the nail files 9 at an end opposite the outlet opening 11. The collar 17 can then be fitted over the first and second housing shells 23, 25. It should be noted that Figure 13 shows the spring 30 to be flat but in reality the spring would be curved with a relatively small radius of curvature.

The present invention is to be understood as defined in the appended claims and is not limited to the specific embodiments described above. Alternative arrangements and suitable materials will be apparent to a reader skilled in the art.

## Claims

1. A nail care device comprising a body (1) provided with a reservoir for a nail care fluid, an applicator (5) for applying the nail care fluid to a nail region, a chamber (7) in the body (1) and a plurality of nail files located in the chamber (7) and removable one at a time therefrom through an outlet opening (11)
wherein the reservoir is formed by a housing (3), detachably connected to the body (1).

2. A nail care device according to claim 1, wherein the housing (3) is connected to the body (1) via a threaded connection.

3. A nail care device according to any of claims 1 or 2, wherein the applicator (5) is detachably connected to the body (1).

4. A nail care device according to claim 3, wherein the applicator (5) and the housing (3) detach as a unit from the body (1).

5. A nail care device according to claim 4, wherein the applicator (5) is detachably connected to the housing (3).

6. A nail care device according to any one of the preceding claims, wherein the reservoir is arranged at one end of the body (1) and the chamber (7) is arranged at the other end of the body (1).

7. A nail care device according to any one of the preceding claims, wherein the applicator (5) comprises a brush.

8. A nail care device according to any one of the preceding claims, wherein the nail care device is a fungal nail care device and the nail care fluid is fungal nail care fluid.

9. A nail care device according to any one of the preceding claims, comprising a dispensing mechanism (21) operable to dispense the nail files one at a time through the outlet opening (11).

10. A device according to claim 9, wherein the dispensing mechanism (21) comprises a biasing means to urge the nail files towards a dispensing position.

11. A device according to claim 9 or 10, wherein the dispensing mechanism (21) comprises an element engageable by a user to cause a nail file (9) to at least partially exit the chamber (7).

12. A device according to claim 11, wherein the element is connected to a pushing means for exerting a force against an end of a nail file (9) located in the dispensing position.

13. A device according to claim 11 or 12, wherein the element is a slide, moveable towards the outlet opening (11).

14. A device according to any one of the preceding claims, wherein at least 5 nail files (9) are located in the chamber (7).

15. A device according to any one of the preceding claims, comprising a blocking means to prevent a user reinserting a nail file (9) through the outlet opening (11) into the chamber (7).

## Patentansprüche

1. Nagelpflegevorrichtung, umfassend einen Körper (1), der mit einem Reservoir für ein Nagelpflegefluid versehen ist, einen Applikator (5) zum Aufbringen des Nagelpflegefluids auf einen Nagelbereich, eine Kammer (7) im Körper (1) und eine Vielzahl von Nagelfeilen, die in der Kammer (7) angeordnet und nacheinander durch eine Auslassöffnung (11) daraus entnehmbar sind,
wobei das Reservoir durch ein Gehäuse (3) gebildet ist, das ablösbar mit dem Körper (1) verbunden ist.

2. Nagelpflegevorrichtung nach Anspruch 1, wobei das Gehäuse (3) über eine Gewindeverbindung mit dem Körper (1) verbunden ist.

3. Nagelpflegevorrichtung nach einem der Ansprüche 1 oder 2, wobei der Applikator (5) ablösbar mit dem Körper (1) verbunden ist.

4. Nagelpflegevorrichtung nach Anspruch 3, wobei der Applikator (5) und das Gehäuse (3) als eine Einheit vom Körper (1) abgelöst werden können.

5. Nagelpflegevorrichtung nach Anspruch 4, wobei der Applikator (5) ablösbar mit dem Gehäuse (3) verbunden ist.

6. Nagelpflegevorrichtung nach einem der vorhergehenden Ansprüche, wobei das Reservoir an einem Ende des Körpers (1) angeordnet ist und die Kammer (7) am anderen Ende des Körpers (1) angeordnet ist.

7. Nagelpflegevorrichtung nach einem der vorhergehenden Ansprüche, wobei der Applikator (5) einen Pinsel umfasst.

8. Nagelpflegevorrichtung nach einem der vorhergehenden Ansprüche, wobei die Nagelpflegevorrichtung eine Pflegevorrichtung für Nagelpilz ist und das Nagelpflegefluid ein Pflegefluid für Nagelpilz ist.

9. Nagelpflegevorrichtung nach einem der vorhergehenden Ansprüche, umfassend einen Ausgabemechanismus (21), der dahingehend betätigbar ist, die Nagelfeilen nacheinander durch die Auslassöffnung (11) auszugeben.

10. Vorrichtung nach Anspruch 9, wobei der Ausgabemechanismus (21) ein Vorspannmittel umfasst, um die Nagelfeilen zu einer Ausgabeposition hin zu drängen.

11. Vorrichtung nach Anspruch 9 oder 10, wobei der Ausgabemechanismus (21) ein Element umfasst, das von einem Benutzer in Eingriff genommen werden kann, um zu veranlassen, dass eine Nagelfeile (9) mindestens teilweise aus der Kammer (7) austritt.

12. Vorrichtung nach Anspruch 11, wobei das Element mit einem Schiebemittel verbunden ist, um eine Kraft gegen ein Ende einer in der Ausgabeposition angeordneten Nagelfeile (9) auszuüben.

13. Vorrichtung nach Anspruch 11 oder 12, wobei das Element ein zu der Auslassöffnung (11) hin beweglicher Schieber ist.

14. Vorrichtung nach einem der vorhergehenden Ansprüche, wobei mindestens 5 Nagelfeilen (9) in der Kammer (7) angeordnet sind.

15. Vorrichtung nach einem der vorhergehenden Ansprüche, umfassend ein Sperrmittel, um zu verhindern, dass ein Benutzer eine Nagelfeile (9) wieder durch die Auslassöffnung (11) in die Kammer (7) einführt.

## Revendications

1. Dispositif de soin des ongles comprenant un corps (1) pourvu d'un réservoir pour un fluide de soin des ongles, un applicateur (5) servant à appliquer le fluide de soin des ongles à une région d'ongle, une chambre (7) dans le corps (1) et une pluralité de limes à ongles situées dans la chambre (7) et pouvant être extraites une à la fois de celle-ci à travers une ouverture de sortie (11),
dans lequel le réservoir est formé par un logement (3), raccordé de manière amovible au corps (1).

2. Dispositif de soin des ongles selon la revendication 1, dans lequel le logement (3) est raccordé au corps (1) par le biais d'un raccordement par vissage.

3. Dispositif de soin des ongles selon l'une quelconque des revendications 1 et 2, dans lequel l'applicateur (5) est raccordé de manière amovible au corps (1).

4. Dispositif de soin des ongles selon la revendication 3, dans lequel l'applicateur (5) et le logement (3) peuvent être séparés du corps (1) d'un seul tenant.

5. Dispositif de soin des ongles selon la revendication 4, dans lequel l'applicateur (5) est raccordé de manière amovible au logement (3).

6. Dispositif de soin des ongles selon l'une quelconque des revendications précédentes, dans lequel le réservoir est disposé à une extrémité du corps (1) et la chambre (7) est disposée à l'autre extrémité du corps (1).

7. Dispositif de soin des ongles selon l'une quelconque des revendications précédentes, dans lequel l'applicateur (5) comprend un pinceau.

8. Dispositif de soin des ongles selon l'une quelconque des revendications précédentes, le dispositif de soin des ongles étant un dispositif de soin antifongique des ongles et le fluide de soin des ongles étant un fluide de soin antifongique des ongles.

9. Dispositif de soin des ongles selon l'une quelconque des revendications précédentes, comprenant un mécanisme de distribution (21) pouvant être mis en oeuvre pour distribuer les limes à ongles une à la fois à travers l'ouverture de sortie (11).

10. Dispositif selon la revendication 9, dans lequel le mécanisme de distribution (21) comprend un moyen de sollicitation servant à solliciter les limes à ongles en direction d'une position de distribution.

11. Dispositif selon la revendication 9 ou 10, dans lequel le mécanisme de distribution (21) comprend un élément avec lequel un utilisateur peut interagir afin de faire sortir au moins partiellement une lime à ongles (9) de la chambre (7).

12. Dispositif selon la revendication 11, dans lequel l'élément est raccordé à un moyen de poussée permettant d'exercer une force contre une extrémité d'une lime à ongles (9) se trouvant dans la position de distribution.

13. Dispositif selon la revendication 11 ou 12, dans lequel l'élément est un coulisseau pouvant être déplacé en direction de l'ouverture de sortie (11).

14. Dispositif selon l'une quelconque des revendications précédentes, dans lequel au moins 5 limes à ongles (9) se trouvent dans la chambre (7).

15. Dispositif selon l'une quelconque des revendications précédentes, comprenant un moyen de blocage servant à empêcher un utilisateur de réinsérer une lime à ongles (9) dans la chambre (7) à travers l'ouverture de sortie (11).
